(19)

Europäisches
Patentamt
European
Patent Office
Office européen
des brevets

(11)  **EP 2 110 125 B1**

(12)  **EUROPEAN PATENT SPECIFICATION**

(45) Date of publication and mention
of the grant of the patent:
**02.03.2011  Bulletin 2011/09**

(51) Int Cl.:
*A61K 9/70* (2006.01)    *A61K 31/565* (2006.01)

(21) Application number: **09158023.3**

(22) Date of filing: **16.04.2009**

(54) **Transdermal drug administration device**

Vorrichtung zur transdermalen Arzneimittelverabreichung

Dispositif d'administration de médicament transdermique

(84) Designated Contracting States:
**AT BE BG CH CY CZ DE DK EE ES FI FR GB GR
HR HU IE IS IT LI LT LU LV MC MK MT NL NO PL
PT RO SE SI SK TR**

(30) Priority: **16.04.2008  JP 2008106314**

(43) Date of publication of application:
**21.10.2009  Bulletin 2009/43**

(73) Proprietor: **Nitto Denko Corporation
Ibaraki-shi
Osaka 567-8680 (JP)**

(72) Inventors:
• **Tamura, Kei
Osaka Osaka 567-8680 (JP)**

• **Inosaka, Keigo
Osaka Osaka 567-8680 (JP)**
• **Saeki, Yuji
Osaka Osaka 567-8680 (JP)**

(74) Representative: **von Kreisler Selting Werner
Deichmannhaus am Dom
Bahnhofsvorplatz 1
50667 Köln (DE)**

(56) References cited:
**DE-A1-102006 026 578    US-A- 4 668 232
US-A- 4 769 028    US-A1- 2005 142 176**

**Description**

Technical Field

The present invention relates to a transdermal drug administration device having a backing layer and a matrix layer provided on at least one surface thereof.

Background Art

A transdermal drug administration device has many advantages such as absorbability of the drug in the gastrointestinal tract, avoidance of first-passage effect in the liver, advantage for people having difficulty in swallowing drugs, prevention of skipped administration and the like. As such, the superiority of the transdermal drug administration device as an administration form has been drawing attention, and various kinds of transdermal drug administration devices have been developed. It has also been proposed to constitute a transdermal drug administration device by combining a plurality of members according to an object. Documents relating to such a transdermal drug administration device are, for example, the following.
There is disclosed a transdermal drug administration device provided with a polymer-blend matrix (patent document 1). The matrix disclosed here is a mere blend of polymers. Thus, the matrix has a homogenized layer. It is difficult for such a device to highly control the drug release.
Furthermore, there is disclosed a transdermal drug administration device having a multi-layer structure (patent documents 2 and 3). However, the adhesive layer in the multi-layer structure of the device disclosed here does not contain a water-absorbing polymer at a particular site, and therefore, a pharmaceutical component in the multi-layer structure may migrate into the adhesive layer due to diffusion or penetration during a long-term storage, resulting in uniform layer constitution of the multi-layer structure. Consequently, a sufficient effect expected from the transdermal drug administration device cannot be achieved.

Prior Art Documents

Patent Documents

    patent document 1: US Patent No. 5,656,286
    patent document 2: JP-A-56-125311
    patent document 3: JP-A-59-207149

US-A-4,668,232 and DE-A-10 2006 026578 disclose a transdermal drug administration device comprising a backing layer and a matrix layer provided on at least one surface of the backing layer, wherein the matrix layer comprises a region located on a proximal side from the backing layer and a region located on a distal side from the backing layer, wherein the first part comprises a water-absorbing polymer in a higher weight concentration than in the second part.

SUMMARY OF THE INVENTION

Problems to be Solved by the Invention

In view of the above-mentioned situation, an object of the present invention is to provide a transdermal drug administration device that enables easy control of drug release.

Means of Solving the Problems

The present inventors have conducted intensive studies and found that a transdermal drug administration device comprising a backing layer and a matrix layer provided on at least one surface of the backing layer, wherein the matrix layer comprises a region located on a proximal side from the backing layer (hereinafter to be referred to as the first part) and a region located on a distal side from the backing layer (hereinafter to be referred to as the second part), at least the first part comprises a water-absorbing polymer, and the water-absorbing polymer in the first part has a higher weight concentration than that of the water-absorbing polymer in the second part, can highly control drug release from the device, which resulted in the completion of the present invention. Accordingly, the present invention provides the following:

[1] A transdermal drug administration device comprising a backing layer and a matrix layer provided on one surface of the backing layer, wherein the matrix layer comprises a region located on a proximal side from the backing layer (hereinafter to be referred to as the first part) and a region located on a distal side from the backing layer (hereinafter to be referred to as the second part), both the first part and the second part comprise a water-absorbing polymer, the water-absorbing polymer is insoluble in the matrix, and the water-absorbing polymer in the first part has a higher weight concentration than that of the water-absorbing polymer in the second part.

[2] The transdermal drug administration device of the above mentioned [1], wherein the weight concentration of the matrix insoluble material in the first part is not less than 1.5-fold of that of the second part and is not more than 10-fold of that of the second part.

[3] The transdermal drug administration device of the above mentioned [1] or [2], wherein the weight concentration of the matrix insoluble material in the first part is 5 - 35 wt% and the weight concentration of the matrix insoluble material in the second part is 1 - 15 wt%.

[4] The transdermal drug administration device of any of the above mentioned [1] to [3], wherein the matrix layer comprises a drug.

[5] The transdermal drug administration device of any of the above mentioned [1] to [4], wherein the matrix layer comprises a material having adhesiveness.

[6] The transdermal drug administration device of any of the above mentioned [1] to [5], wherein the matrix comprises at least the first layer comprising the first part and the second layer having the second part.

[7] The transdermal drug administration device of any of the above mentioined [1] to [6], further comprising a release liner laminated on the skin contact surface of the matrix layer.

Effect of the Invention

According to the transdermal drug administration device of the present invention, drug release can be highly controlled. Particularly, by adjusting the weight concentration of the water-absorbing polymer in the first part and that of the water-absorbing polymer in the second part, drug release from the device can be freely controlled. Therefore, the weight concentration of other components in the matrix layer, for example, a drug and the like, at one site in the matrix layer does not need to be controlled. As a result, flexible design of the transdermal drug administration device becomes possible and the drug can be stably released from the device. When the water-absorbing polymer is insoluble in the matrix, the polymer does not easily migrate from one site even when the device is stored for a long period, since the polymer is not dissolved in the matrix layer. Therefore, release of the drug from the device can be controlled even after a long-term storage of the device.

Brief Description of the Drawings

Fig. 1 shows a schematic sectional view of one embodiment of the transdermal drug administration device.
Fig. 2 shows a schematic sectional view of one embodiment of the transdermal drug administration device.
Fig. 3 is a graph showing the results of Experimental Example 1.

Mode for Practicing the Invention

The present invention is explained in detail in the following.
The transdermal drug administration device of the present invention has a matrix layer on at least one surface of a backing layer. The matrix layer comprises at least a drug, a water-absorbing polymer, and other material having adhesiveness.
The "material having adhesiveness" is not particularly limited as long as it can conveniently bind each member constituting the device, and causes adhesion of the matrix layer to the skin. A material having adhesiveness at ambient temperature (25°C) is preferable, since it can conveniently bind each member constituting the device, and cause adhesion of the matrix layer to the skin. Generally, one containing a polymer, i.e., an adhesive, can be used. The adhesive here means an elastomer having adhesiveness by itself, or a polymer composition having adhesiveness, comprising an elastomer and the below-mentioned tackifier. While the amount of the adhesive in the matrix layer is not particularly limited, it is preferably 60 - 90 wt%, more preferably 70 - 85 wt%. When the amount is not less than 60 wt%, sufficient adhesion strength of the matrix layer to the skin is ensured. When the amount is not more than 90 wt%, flexible design of the matrix layer is ensured.

Examples of such adhesive include acrylic adhesives comprising an acrylic polymer; rubber adhesives including rubber elastomers such as styrene-diene-styrene block copolymers (e.g., styrene-isoprene-styrene block copolymer, styrene-butadiene-styrene block copolymer and the like), polyisoprene, polyisobutylene, polybutadiene and the like; silicone adhesives including silicone elastomers such as silicone rubber, dimethylsiloxane base, diphenylsiloxane base and the like; vinylether adhesives such as polyvinyl methylether, polyvinyl ethylether, polyvinyl isobutylether and the like; vinylester adhesives such as vinyl acetate-ethylene copolymer and the like; polyester adhesives made of a carboxylic acid component (e.g., dimethylterephthalate, dimethylisophthalate, dimethylphthalate and the like) and a polyvalent alcohol component (e.g., ethyleneglycol and the like). They may be used alone or in a mixture of two or more kinds thereof. From the aspects of skin adhesiveness, hydrophobic adhesives and water-free adhesives are preferable. From the aspects of balance of easy availability, adhesive property, stability and safety, rubber adhesives including polyisobutylene are more preferable.

When a tackifier is contained in an adhesive, examples of the tackifier include polybutenes, petroleum resins (e.g., aromatic petroleum resin, aliphatic petroleum resin), terpene resin, rosin resin, coumaroneindene resin, styrene resins (e.g., styrene resin, α-methylstyrene), hydrogenated petroleum resins (e.g., alicyclic saturated hydrocarbon resin) and the like. Among these, polybutenes are preferable since the storage stability of the drug is fine. One or more kinds of tackifiers can be used in combination.

The amount of the tackifier is preferably 30 - 90 wt%, more preferably 50 - 70 wt%, of the total weight of the adhesive. When the amount of the tackifier is less than 30 wt%, tackiness may become poor, and when it exceeds 90 wt%, the adhesive layer becomes stiff and the skin adhesiveness tends to decrease.

In the following, when polyisobutylene is contained in a matrix layer, polyisobutylene having cohesiveness necessary for a matrix layer and essentially used is referred to as the first polyisobutylene, and polyisobutylene further added for various purposes such as enhancement of the adhesive force of the matrix layer and the like is referred to as the second polyisobutylene.

While the first polyisobutylene is not particularly limited, one having a viscosity average molecular weight of 1,600,000 - 6,500,000 is preferable, 2,000,000 - 6,000,000 is more preferable, and 3,000,000 - 5,000,000 is most preferable. When the viscosity average molecular weight is less than 1,600,000, the cohesive strength of the matrix layer may decrease, possibly causing an adhesive residue by detachment. When it exceeds 6,500,000, compatibility with other components decreases and uniformity as an adhesive may not be maintained, possibly causing an adhesive residue on the skin when the device is detached from the skin.

When the second polyisobutylene is added to strengthen the adhesive force of the matrix layer, the second polyisobutylene preferably has a viscosity average molecular weight of 30,000 - 100,000, more preferably 40,000 - 80,000, most preferably 50,000 - 60,000. When it is less than 30,000, the amount to be added may be limited, since it weakens the cohesive strength. When it exceeds 100,000, the second polyisobutylene has a molecular weight almost the same as that of the first polyisobutylene, which may cause difficulty in exhibiting an adhesive force-improving effect.

While the proportion of the weight (a) of the second polyisobutylene relative to the weight (b) of the first polyisobutylene, $(a/b) \times 100$ [wit%], is not particularly limited, it is preferably 50 - 200 wt%, more preferably 75 - 150 wt%, most preferably 90 - 110 wt%. When the proportion of the second polyisobutylene is not less than 50 wt%, the effect of the second polyisobutylene is sufficiently exhibited. When the above-mentioned proportion is not more than 200 wt%, sufficient cohesiveness of the matrix layer is ensured.

When the second polyisobutylene is added to further enhance the cohesive strength of the matrix layer, the second polyisobutylene preferably has a higher viscosity average molecular weight than that of the first polyisobutylene.

The viscosity average molecular weight in the present specification is obtained by calculating the Staudinger index ($J_0$) from the capillary flow time of Uberode-type viscometer at 20°C by the Schulz-Blaschke equation, and determining from the following equations using the Jo value:
(Equations)

$$J_0 = \eta_{sp}/c\,(1+0.31\eta_{sp})\ cm^3/g\ \ (Schulz\text{-}Blaschke\ equation)$$

$$\eta_{sp} = t/t_0 - 1$$

t: flow time of solution (by Hagenbach-couette correction)

$t_0$: flow time of solvent (by Hagenbach-couette correction)

c: concentration ($g/cm^3$) of solution

$J_0 = 3.06 \times 10^{-2} Mv^{0.65}$

Mv: viscosity average molecular weight

In the present specification, the water-absorbing polymer means a polymer material that absorbs water in an amount of at least its weight or more, and swells. While the water-absorbing polymer is not particularly limited, polyvinyl alcohol, polyvinylpyrrolidone, crosslinked polyvinylpyrrolidone, methoxyethylenemaleic anhydride copolymer, methacrylic acid polymer or polysaccharide sodium alginate, ammonium alginate, carboxymethylcellulose, sodium carboxymethylcellulose, hydroxymethylcellulose, hydroxypropylcellulose, hydroxypropylethylcellulose, methylcellulose, soluble starch, carboxymethylamylose, dextrin etc., other pectin, hemicellulose such as agar powder and the like, plant rubbers such as gum Arabic, tragacanth rubber, xanthan gum and the like, a starch grafted acrylate and the like can be mentioned. They may be used alone or in a mixture of two or more kinds thereof.

The water-absorbing polymer is insoluble in the matrix even after water absorption. The water-absorbing polymer insoluble in the matrix is hereinafter to be referred to as a "matrix layer-insoluble material". The matrix layer-insoluble material is a component other than a drug, which is not dissolved in a matrix layer at ambient temperature (25°C) but dispersed in a solid state. Since the matrix layer insoluble material is insoluble in a matrix layer, it does not easily migrate in the matrix. As a result, the weight concentration of the matrix layer-insoluble material in the matrix layer does not change easily even after a long-term storage of the device. The matrix layer-insoluble material swells by absorption of water such as sweat and the like, and provides an effect to release the drug from the matrix layer. This effect becomes higher as the matrix layer-insoluble material has a higher weight concentration, thus resulting in a faster drug release rate. Since the weight of the matrix layer-insoluble material does not change, stable release property can be maintained even after a long-term storage. For example, the release rate of a drug from a particular site of a matrix layer, where the weight concentration of the matrix layer-insoluble material is higher than in other site, is faster than that of other site. Particularly, when the matrix layer-insoluble material absorbs water, this tendency becomes marked, and the present invention can be advantageously practiced in this event.

The matrix layer-insoluble material is not particularly limited as long as it is substantially insoluble in the matrix (being substantially insoluble means that the matrix insoluble material itself is insoluble in the matrix but may become slightly soluble due to heat history during production of the device, decomposition under severe conditions such as a long-term storage of the device and the like, and the like), water-absorbing one is preferable for the above-mentioned reasons. From the above aspects, the material is preferably crosslinked polyvinylpyrrolidone.

Crosslinked polyvinylpyrrolidone can be obtained by copolymerization of N-vinyl-2-pyrrolidone and a multifunctional monomer. Examples of the multifunctional monomer to be used include di(meth)acrylates such as hexamethylene glycol di(meth)acrylate, ethylene glycol di(meth)acrylate, triethylene glycol di(meth)acrylate and the like; tri(meth)acrylates such as trimethylolpropanetri(meth)acrylate and the like; and tetra(meth)acrylate such as pentaerythritoltetra (meth)acrylate and the like; polyallyl compounds such as diethylene glycol bisallylcarbonate, triallylglycerol, triallylcyanurate and the like; polymaleimide compounds such as ethylenebismaleimide and the like; and the like. Alternatively, divinylbenzene, methylenebisacrylamide, ethylidenebisvinylpyrrolidone, divinylketone, butadiene, isoprene and the like can also be used.

The amount of the multifunctional monomer to be copolymerized is preferably 0.1 - 10 mol% of the total amount of the monomer. When it is less than 0.1 mol%, the obtained crosslinked polyvinylpyrrolidone dissolves or swells in the elastomer, possibly making it difficult to function as a crosslinked form. When the amount of the multifunctional monomer exceeds 10 mol%, the property of vinylpyrrolidone may be diluted and cannot be exhibited easily.

Crosslinked polyvinylpyrrolidone is commercially available with trade names of Kollidon CL, Kollidon CL-M (both manufactured by BASF), Polyplasdone (manufactured by ISB), crospovidone (manufactured by GOKYO TRADING CO., LTD.) and the like. In consideration of its effect relative to the amount of addition, one having a small particle size has the widest surface area and is effective. From such aspects, preferred is Kollidon CL-M.

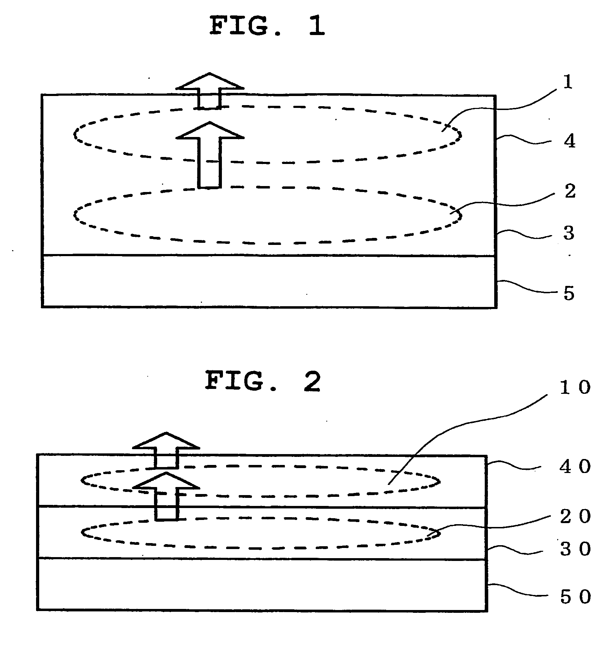

Fig. 1 is a schematic sectional view of the transdermal administration device of the present invention. In Fig. 1, 1 is a matrix layer-insoluble material of the second part, 2 is a matrix layer-insoluble material of the first part, 3 is the first part, 4 is the second part, 5 is a backing layer and an arrow shows the direction of drug release. The difference in the arrow sizes shows a difference in the drug release rate, wherein a large arrow means a faster drug release rate as compared to a small arrow.

Fig. 1 is now explained in more detail. The matrix layer includes the first part and the second part. The first part and the second part contain a matrix layer-insoluble material, and the weight concentration of the first part is higher than that of the second part. The release rate of the drug from the first part is faster than that from the second part. The first part is located closer to the backing layer than is the second part. In a general transdermal administration device, a drug is rapidly released in the initial stages of transdermal administration, after which the release amount of the drug gradually decreases, and consequently, the drug is not stably administered transdermally in some cases. In the present invention, however, since the second part having a low weight concentration of the matrix layer insoluble component is present near the skin contact surface, the drug release rate from the skin contact surface of the matrix layer does not become too high in the initial stages of transdermal administration, whereby the risk of side effects due to an administration of an excess amount of the drug can be reduced.

The matrix insoluble material is contained in both the first part and the second part.

To achieve similar drug release rates in the early stages of transdermal administration and the later stages of transdermal administration, the weight concentration of the matrix insoluble material in the first part is preferably not less than 1.5-fold, more preferably not less than 2.0-fold, most preferably not less than 2.5-fold of that of the second part. While the upper limit of the ratio is not particularly limited, for a matrix insoluble material to be stably maintained in a matrix layer, it is preferably not more than 10-fold.

The weight concentration of the matrix insoluble material in the first part is generally 5 - 35 wt%, preferably 10 - 30 wt%, more preferably 15 - 25 wt%. A concentration of not less than 5 wt% is advantageous for the retention of a high polar organic liquid component, and a concentration of not more than 35 wt% is advantageous for the anchor property to a backing layer.

The weight concentration of the matrix insoluble material in the second part is generally 1 - 15 wt%, preferably 3 - 12 wt%, more preferably 5 - 10 wt%. A concentration of not less than 1 wt% is advantageous for drug releaseability, and a concentration of not more than 15 wt% is advantageous for skin adhesion.

The drug is not particularly limited, and a drug that can be administered to an animal (e.g., mammals such as human and the like) through the skin, i.e., transdermally absorbable drug, is preferable. Specific examples of the drug include general anesthetics, hypnotic sedatives, antiepileptic drugs, antipyretic analgesic antiphlogistic drugs, anti-vertigenous drugs, psychoneurotic drugs, topical anesthetics, skeleton muscle relaxants, autonomic drugs, antispasmodic drugs, anti-parkinsonian drugs, anti-histamine drugs, cardiac stimulants, drugs for arrhythmia, diuretic, hypotensive drug, vasoconstrictor, coronary vasodilator, peripheral vasodilators, arteriosclerosis drugs, drugs for circulatory organ, anapnoics, antitussive expectorant, hormone drugs, external drugs for mattery diseases, analgesic-antipruritic-styptic-antiphlogistic drugs, drugs for parasitic dermatic diseases, drugs for arrest of bleeding, gout treatment drugs, drugs for diabetes, drugs for anti-malignant tumor, antibiotic, chemical therapy drugs, narcotic, quit smoking aids and the like.

The drug content is not particularly limited as long as the effect of the transdermally absorbable drug is afforded and the adhesive property of an adhesive is not impaired. It is preferably 0.1 - 10 wt%, more preferably 0.1 - 7 wt%, of an adhesive. When it is less than 0.1 wt%, the treatment effect may not be sufficient, and when it is more than 10 wt%, skin irritation may be developed and the content may be economically disadvantageous.

When the matrix layer-insoluble material is a water-absorbing polymer, a hydrophilic drug is preferable. The hydrophilic drug here has a coefficient of partition (1-octanol/water), i.e., $\log P_{ow}$, of 0.5 - 5.5. To sufficiently achieve the effect of the present invention, the $\log P_{ow}$ of the drug is preferably 1.0 - 5.0, more preferably 3.0 - 5.0. When the $\log P_{ow}$ of the drug is less than 0.5, the drug has high hydrophilicity. Even with the present invention, such drug may be precipitated as a crystal in the matrix layer. On the other hand, when the $\log P_{ow}$ of the drug exceeds 5.5, the drug has high hydrophobicity and the drug less likely precipitates as a crystal in the matrix layer. In this case, the benefit of the present invention is not very high.

The $\log P_{ow}$ here is an index showing the hydrophilicity or hydrophobicity of the compound, which is calculated using a $\log P_{ow}$ calculation software Cache (registered trade mark) manufactured by FUJITSU LIMITED. For measurement (calculation) of $\log P_{ow}$, the structural formula of the compound is input into the aforementioned calculation software and $\log P_{ow}$ is calculated.

When the drug is solid at ambient temperature, namely, a drug having a melting point of not less than 100°C is advantageous in the present invention. While the upper limit of the melting point is not particularly limited, it is practically preferably not more than 1000°C.

The melting point of the drug here means a value measured by the following method.
apparatus: melting point measurement apparatus manufactured by MIYAMOTO RIKEN IND JAPAN
measurement method: According to the Japanese Pharmacopoeia, 15th Edition, melting point measurement method, First method, the indication of the thermometer at the time point when a sample is liquefied in a capillary tube and a solid is not at all observed is read and taken as a melting point.

It is advantageous to form a first layer containing the first part and a second layer containing the second part in the matrix layer, whereby the drug release rate from the skin contact surface can be highly controlled.

Fig. 2 shows a schematic cross section of this embodiment of the present invention. In this embodiment, the matrix layer consists of a first layer (30) and a second layer (40). It is also possible to further form a third layer and the like. The first layer (30) containing a first part (20) of a matrix layer is laminated on one surface of a backing layer (50), and the second layer (40) containing a second part (10) of the matrix layer is laminated thereon. The first layer and the second layer contain a matrix layer insoluble component, and the first layer contains a matrix layer insoluble component in an amount equal to, preferably more than, the amount of a matrix layer insoluble component contained in the second layer. In this connection, the aforementioned weight concentration is applicable. In this embodiment, since the first layer consists of the first part and the second layer consists of the second part, the aforementioned drug release rate is certainly controlled, thus enabling stabilization of the drug release rate.

While the composition of the first layer and the second layer is not particularly limited, the drug concentrations thereof are preferably the same so as to stabilize the quality of the device, since the drug may move during the production step of the device, during storage thereof and the like due to equilibration. For the same reason, the mixing ratio of materials having adhesiveness in the first layer and the second layer is preferably the same. Particularly, since a drug having a high concentration in the matrix layer may be separated (crystallize/bleed) from the matrix layer, changing the concentration requires careful consideration. In the present invention, since the concentrations of the water-absorbing polymers in the first layer and the second layer are different, the amounts of the materials having adhesiveness in the first layer and the second layer are preferably changed only in the amount corresponding to the concentration of the water-absorbing polymer.

When desired, the matrix layer can contain an organic liquid component as a material having adhesiveness. The organic liquid component can plasticize a matrix layer, adjust an adhesive power to the adhesion site, and control transdermal absorbability of a drug that can be contained in the matrix layer.

Examples of the organic liquid component include plasticizers such as diisopropyladipate, diacetylsebacate and the like; glycols such as ethylene glycol, diethylene glycol, propylene glycol, triethylene glycol, polyethylene glycol, polypropylene glycol and the like, fats and oils such as olive oil, castor oil, squalane, lanolin and the like, and the like; hydrocarbons such as liquid paraffin and squalane; various surfactants; alcohols such as polyvalent alcohols (e.g., glycerol and the like), or monoalcohols (e.g., octyldodecanol, oleyl alcohol, ethoxylated stearyl alcohol and the like), and the like; oleic acid monoglyceride, caprylic acid monoglyceride; glycerol monoesters such as lauryl acid monoglyceride or glycerol esters such as glycerol diester, glycerol triester and mixtures thereof; fatty acid esters such as ethyl laurate, isopropyl myristate, isotridecyl myristate, octyl palmitate, isopropyl palmitate, ethyl oleate and diisopropyl adipate; fatty acids such as oleic acid and caprylic acid; as well as N-methylpyrrolidone, 1,3-butanediol and the like. Among these, from the aspect of transdermal absorbability of physiologically active component, alcohols, glycerol esters and fatty acid esters are preferable.

While the amount of the organic liquid component in the matrix layer is not particularly limited, the total weight thereof is preferably 5 - 25 wt%, more preferably 10 - 20 wt%, relative to the total weight of the adhesive layer. When the amount is not less than 5 wt%, sufficient adhesion strength of the matrix layer to the skin is ensured. When the amount is not more than 20 wt%, the cohesive strength of the matrix layer can be ensured, whereby the flexibility of the design of the matrix layer is increased.

While the backing layer is not particularly limited, one substantially impermeable to a drug and the like is preferable; in other words, one that does not permit a decrease in the content of a drug, an additive and the like, which are active ingredients in the matrix layer, by preventing them from being lost from the back face through the backing layer.

Examples of the backing layer include single films or laminate films of polyester, nylon, saran (registered trade mark), polyethylene, polypropylene, polyvinyl chloride, ethylene-ethyl acrylate copolymer, polytetrafluoroethylene, Surlyn (registered trade mark), metal foil and the like, and the like. The thickness of the backing layer is generally 10 - 500 $\mu$m, preferably 10 - 200 $\mu$m.

The backing layer is preferably a laminate film of a nonporous plastic film and a porous film, which are made from the above-mentioned materials, to improve an adhesion force (anchor force) between the backing layer and the matrix layer. In this case, the matrix layer is preferably formed on the porous film side.

As such porous film, one capable of improving the anchor force to a matrix layer is employed. Specific examples include paper, woven fabric, non-woven fabric, knitted fabric, mechanically perforated sheet and the like. Among these, paper, woven fabric and non-woven fabric are particularly preferable from the aspects of handling property.

A porous film having a thickness of 10 - 200 $\mu$m is employed in consideration of improvement of anchor force, flexibility of transdermal drug administration device as a whole and adhesion operability and the like. For a thin preparation such as a plaster type or an adhesive tape type, one having a thickness of 10 - 100 $\mu$m is employed.

When a woven fabric or a non-woven fabric is used as a porous film, the fabric weight is preferably set to 5 - 30 g/m$^2$, more preferably 6 - 15 g/m$^2$. In the present invention, the most preferable backing layer is a laminate film of a 1.5 - 6 $\mu$m thick polyester film (preferably poly(ethylene terephthalate) film) and a polyester (preferably poly(ethylene terephthalate)) non-woven fabric having a fabric weight of 6 - 12 g/m$^2$.

The transdermal drug administration device of the present invention preferably has a release liner laminated on the skin contact surface of a matrix layer so as to protect said surface until use.

The release liner is not particularly limited as long as sufficiently light release property can be ensured. Examples thereof include films of polyester, polyvinyl chloride, polyvinylidene chloride, poly(ethylene terephthalate) and the like, high quality paper, glassine paper and the like, laminate films of polyolefin and paper such as high quality paper, glassine paper and the like, and the like, whose face to be in contact with a matrix layer underwent a peeling treatment by applying a silicone resin, a fluororesin and the like. The thickness of the release liner is generally 10 - 200 $\mu$m, preferably 25 - 100 $\mu$m.

The release liner of the present invention is preferably made of a polyester (particularly poly(ethylene terephthalate)) resin from the aspects of barrier property and cost. Furthermore, in view of handling property, a release liner having a thickness of about 25 - 100 μm is more preferable.

The device may take the form of a sheet or a tape.

The transdermal drug administration device of the present invention, which is free of a multi-layer structure, can be produced by a method including mixing a water-absorbing polymer, a drug, a material having adhesiveness at an ambient temperature and other starting materials to give a composition for matrix layer formation, and laminating the composition on a liner or a backing layer. For example, a centrifugal force is applied to a direction approximately perpendicular to the backing layer using a centrifuge to unevenly distribute the water-absorbing polymer in the backing layer, whereby the device can be produced.

In addition, the transdermal drug administration device of the present invention, which has a multi-layer structure, can be produced by a method including the following: an adhesive, a water-absorbing polymer, a drug and an organic liquid component are dispersed or dissolved in a solvent, the resulting blend for the first part is applied onto a liner, the liner is dried and applied to a liner material., a blend for the second part is separately applied onto a liner, and the liner is dried and applied to the first part sheet produced earlier.

Examples

The present invention is explained in more detail in the following by referring to Examples, which are not to be construed as limitative. In the following, "parts" means "parts by weight".

[1] Preparation of composition A for matrix layer formation

To a mixture of polyisobutylene A (16.3 parts, viscosity average molecular weight of 4,000,000), polyisobutylene B (16.3 parts, viscosity average molecular weight 55,000), polybutene (48.8 parts) as a tackifier, dipropylene glycol (1.8 parts), propyleneglycol monolaurate (1.5 parts), oleyl alcohol (5 parts), isopropyl myristate (5 parts) and crosslinked polyvinylpyrrolidone (Kollidon CL-M, 5 parts) was added a suitable amount of n-hexane, and the mixture was mixed with estradiol (0.3 part) to give composition A for matrix layer formation.

[2] Preparation of composition B for matrix layer formation

To a mixture of polyisobutylene A (15.3 parts), polyisobutylene B (15.3 parts), the aforementioned tackifier (45.8 parts), dipropylene glycol (1.8 parts), propyleneglycol monolaurate (1.5 parts), oleyl alcohol (5 parts), isopropyl myristate (5 parts) and crosslinked polyvinylpyrrolidone (Kollidon CL-M, 10 parts)) was added n-hexane, and the mixture was further mixed with estradiol (0.3 part) to give composition B for matrix layer formation.

[3] Preparation of composition C for matrix layer formation

To a mixture of polyisobutylene A (14.3 parts), polyisobutylene B (14.3 parts), the aforementioned tackifier (42.8 parts), dipropylene glycol (1.8 parts), propyleneglycol monolaurate (1.5 parts), oleyl alcohol (5 parts), isopropyl myristate (5 parts) and crosslinked polyvinylpyrrolidone (Kollidon CL-M, 15 parts)) was added n-hexane, and the mixture was further mixed with estradiol (0.3 part) to give composition C for matrix layer formation.

The parts by weight of respective components except n-hexane in the above-mentioned composition A for matrix layer formation, composition B for matrix layer formation and composition C for matrix layer formation are collectively shown in Table 1.

Table 1

| | composition A for matrix layer formation | composition B for matrix layer formation | composition C for matrix layer formation |
|---|---|---|---|
| estradiol | 0.3 | 0.3 | 0.3 |
| crosslinked polyvinylpyrrolidone | 5 | 10 | 15 |
| dipropylene glycol | 1.8 | 1.8 | 1.8 |
| propyleneglycol monolaurate | 1.5 | 1.5 | 1.5 |

(continued)

| | composition A for matrix layer formation | composition B for matrix layer formation | composition C for matrix layer formation |
|---|---|---|---|
| oleyl alcohol | 5 | 5 | 5 |
| isopropyl myristate | 5 | 5 | 5 |
| polyisobutylene A | 16.3 | 15.3 | 14.3 |
| polyisobutylene B | 16.3 | 15.3 | 14.3 |
| tackifier | 48.8 | 45.8 | 42.8 |

Example 1

Composition C for matrix layer formation was applied to a polyester film (75 $\mu$m thick) such that the thickness after drying was 80 $\mu$m, dried and applied to a polyester film (12 $\mu$m thick). Moreover, composition A for matrix layer formation was applied to a polyester film (75 $\mu$m thick) such that the thickness after drying was 80 $\mu$m, and applied to the dried film to give a 160 $\mu$m thick transdermal drug administration device.

Comparative Example 1

Composition B for matrix layer formation was applied to a polyester film (75 $\mu$m thick) such that the thickness after drying was 80 $\mu$m, dried and applied to a polyester film (12 $\mu$m thick). Moreover, composition B for matrix layer formation was applied to a polyester film (75 $\mu$m thick) such that the thickness after drying was 80 $\mu$m, and applied to the dried film to give a 160 $\mu$m thick transdermal drug administration device.

Experimental Example 1

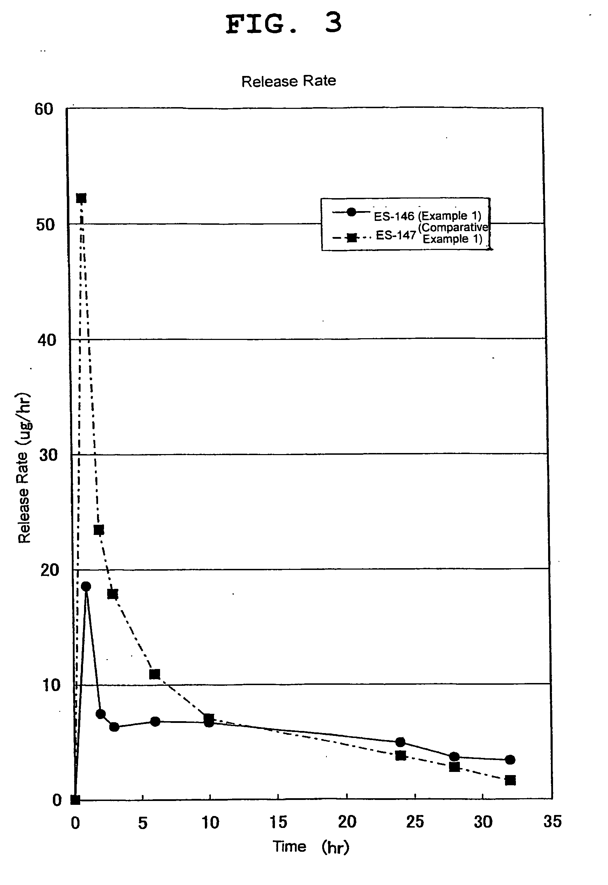

An adhesive agent release test was performed according to U.S. Pharmacopeia 26, <724> Drug Release, Transdermal Delivery Systems-General Drug Release Standards. The solutions released in 1, 2, 3, 6, 10, 24, 28, 32, 48, 52, 56, 72, 76, 80 hr from the start of the test were recovered. The solutions were filtered through membrane filter, quantified by high performance liquid chromatography (HPLC) and the amount of the released estradiol was determined. The release rate was calculated from the amount of estradiol released in a predetermined time to the content of estradiol in the test adhesive agent. The results are shown in Fig. 3.

It is clear from Fig. 3 that the drug initial release rate was suppressed in Example 1 (Fig. 3, ES-146) of the present invention as compared to Comparative Example 1 (Fig. 3, ES-147). In addition, it is clear that the drug was stably released from the device even after a long time.

Explanation of Symbols

1      matrix layer-insoluble material of second part
2      matrix layer-insoluble material of first part
3      first part
4      second part
5      backing layer
10     matrix layer-insoluble material of second part
20     matrix layer-insoluble material of first part
30     first layer of matrix layer
40     second layer of matrix layer
50     backing layer

**Claims**

1. A transdermal drug administration device comprising a backing layer and a matrix layer provided on one surface of the backing layer, wherein the matrix layer comprises a region located on a proximal side from the backing layer (hereinafter to be referred to as the first part) and a region located on a distal side from the backing layer (hereinafter to be referred to as the second part), both the first part and the second part comprise a water-absorbing polymer, the water-absorbing polymer is insoluble in the matrix, and the water-absorbing polymer in the first part has a higher weight concentration than that of the water-absorbing polymer in the second part.

2. The transdermal drug administration device of claim 1, wherein the weight concentration of the matrix insoluble material in the first part is not less than 1.5-fold of that of the second part and is not more than 10-fold of that of the second part.

3. The transdermal drug administration device of claim 1 or 2, wherein the weight concentration of the matrix insoluble material in the first part is 5 - 35 wt% and the weight concentration of the matrix insoluble material in the second part is 1 - 15 wt%.

4. The transdermal drug administration device of any of claims 1 to 3, wherein the matrix layer comprises a drug.

5. The transdermal drug administration device of any of claims 1 to 4, wherein the matrix layer comprises a material having adhesiveness.

6. The transdermal drug administration device of any of claims 1 to 5, wherein the matrix comprises at least the first layer comprising the first part and the second layer having the second part.

7. The transdermal drug administration device of any of claims 1 to 6, further comprising a release liner laminated on the skin contact surface of the matrix layer.

**Patentansprüche**

1. Vorrichtung zur transdermalen Wirkstoffverabreichung, die eine Trägerschicht und eine auf einer Fläche der Trägerschicht befindliche Matrixschicht umfasst, wobei die Matrixschicht einen Bereich, der sich auf der zur Trägerschicht proximalen Seite befindet (im Folgenden als erster Teil bezeichnet), und einen Bereich, der sich auf der von der Trägerschicht distalen Seite befindet (im Folgenden als zweiter Teil bezeichnet), umfasst, sowohl der erste Teil als auch der zweite Teil ein wasserabsorbierendes Polymer umfassen, das wasserabsorbierende Polymer in der Matrix unlöslich ist und das wasserabsorbierende Polymer im ersten Teil eine höhere Gewichtskonzentration hat als das wasserabsorbierende Polymer im zweiten Teil.

2. Vorrichtung zur transdermalen Wirkstoffverabreichung gemäß Anspruch 1, wobei die Gewichtskonzentration des in der Matrix unlöslichen Materials im ersten Teil nicht geringer ist als das 1,5-fache des zweiten Teils und nicht größer ist als das 10-fache des zweiten Teils.

3. Vorrichtung zur transdermalen Wirkstoffverabreichung gemäß Anspruch 1 oder 2, wobei die Gewichtskonzentration des in der Matrix unlöslichen Materials im ersten Teil 5-35 Gew.-% beträgt und die Gewichtskonzentration des in der Matrix unlöslichen Materials im zweiten Teil 1-15 Gew.-% beträgt.

4. Vorrichtung zur transdermalen Wirkstoffverabreichung gemäß einem der Ansprüche 1 bis 3, wobei die Matrixschicht einen Wirkstoff umfasst.

5. Vorrichtung zur transdermalen Wirkstoffverabreichung gemäß einem der Ansprüche 1 bis 4, wobei die Matrixschicht ein Material mit Klebeeigenschaft umfasst.

6. Vorrichtung zur transdermalen Wirkstoffverabreichung gemäß einem der Ansprüche 1 bis 5, wobei die Matrix wenigstens die erste Schicht mit dem ersten Teil und die zweite Schicht mit dem zweiten Teil umfasst.

7. Vorrichtung zur transdermalen Wirkstoffverabreichung gemäß einem der Ansprüche 1 bis 6, die weiterhin eine Trennschicht umfasst, die auf die Hautkontaktfläche der Matrixschicht laminiert ist.

**Revendications**

1. Dispositif d'administration de médicament transdermique comprenant une couche de support et une couche de matrice ménagée sur une surface de la couche de support, dans lequel la couche de matrice comprend une zone située sur un côté proximal de la couche de support (ci-après dénommée la première partie) et une zone située sur un côté distal de la couche de support (ci-après dénommée la seconde partie), la première partie et la seconde partie comprenant toutes deux un polymère absorbant l'eau, le polymère absorbant l'eau étant insoluble dans la matrice, et le polymère absorbant l'eau dans la première partie ayant une concentration en poids supérieure à celle du polymère absorbant l'eau dans la seconde partie.

2. Dispositif d'administration de médicament transdermique selon la revendication 1, dans lequel la concentration en poids du matériau insoluble dans la matrice dans la première partie n'est pas inférieure à 1,5 fois celle de la seconde partie et ne dépasse pas 10 fois celle de la seconde partie.

3. Dispositif d'administration de médicament transdermique selon la revendication 1 ou 2, dans lequel la concentration en poids du matériau insoluble dans la matrice, dans la première partie, est de 5-35 % en poids et la concentration en poids du matériau insoluble dans la matrice, dans la seconde partie, est de 1-15 % en poids.

4. Dispositif d'administration de médicament transdermique selon l'une quelconque des revendications 1 à 3, dans lequel la couche de matrice comprend un médicament.

5. Dispositif d'administration de médicament transdermique selon l'une quelconque des revendications 1 à 4, dans lequel la couche de matrice comprend un matériau doté d'une adhésivité.

6. Dispositif d'administration de médicament transdermique selon l'une quelconque des revendications 1 à 5, dans lequel la matrice comprend au moins la première couche comprenant la première partie et la seconde couche ayant la seconde partie.

7. Dispositif d'administration de médicament transdermique selon l'une quelconque des revendications 1 à 6, comprenant en outre un revêtement protecteur de libération stratifié sur la surface de contact avec la peau de la couche de matrice.

FIG. 1

FIG. 2

# FIG. 3

Release Rate

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5656286 A
- JP 56125311 A
- JP 59207149 A
- US 4668232 A
- DE 102006026578 A